# EUROPEAN PATENT APPLICATION

(11) **EP 2 392 670 A1**
(43) Date of publication of application: **07.12.2011**
(21) Application number: 11004456.7
(22) Date of filing: 31.05.2011
(51) Int. Cl.: C12Q 1/68

(54) **Method for detecting nucleic acid in biological sample**

(30) Priority: 03.06.2010 JP 2010127902
(71) Applicant: Olympus Corporation, Shibuya-ku Tokyo 151-0072 (JP)
(72) Inventor: Tanigami, Yasuo, Tokyo (JP)
(74) Representative: Schicker, Silvia

(57) **Abstract**

The present invention is to provide a method capable of efficiently and readily detecting a nucleic acid from a biological sample such as feces.

The present invention provides a method for detecting a nucleic acid in a biological sample, comprising: (A) a step of mixing a biological sample with a nucleic acid stabilizer solution comprising a water-soluble organic solvent as an active ingredient, and thereafter preserving the thus yielded suspension for a predetermined period of time; and (B) a step of performing a reverse transcription reaction and/or a nucleic acid amplification reaction with use of a part of the suspension yielded from the step (A) as a template solution, to thereby detect a nucleic acid in the suspension.

## Description

### Field of the Invention

The present invention relates to a method for readily detecting a nucleic acid in a biological sample with sufficient precision.

### Description of Related Art

With recent developments of gene analysis technologies, attempts have been made to analyze and make use of a nucleic acid in a biological sample for diagnosis, treatment, and such measures against diseases. The use of a nucleic acid in a biological sample such as feces or blood has advantages in that the invasiveness is lower and the burden on patients is smaller as compared to other clinical tests such as endoscopy. In addition, because disease-related genes are directly tested by nucleic acid analyses, another advantage is that highly reliable results can be obtained. For example, early detection of cancer, or investigation of the degree of advancement can be conducted by checking the presence or absence of a cancer cell in feces or blood, or the presence or absence of a cancer cell-derived gene.

Regarding the method for detecting a nucleic acid contained in feces, for example, Patent Document 1 discloses a method in which untreated intact feces are supplied to a DNA amplification method thereby detecting a target nucleic acid in the feces. This method can enable easy checking of the presence or absence of infection with pathogenic bacteria such as cholera bacteria. However, since diverse and various types of substances other than nucleic acids are also contained in a biological sample such as feces or blood, a problem arises in that the nucleic acids are quite susceptible to decomposition. For this reason, when untreated intact feces are used, nucleic acids may be damaged due to decomposition or the likes before the DNA amplification reaction; in which case, no cancer cell-derived nucleic acid may be detected or false-negative results may be shown although in fact cancer cells are contained in the biological sample. Accordingly, in order to detect a cancer cell-derived nucleic acid which is contained at only a very small amount in a biological sample, it is important to take a measurement to prevent decomposition of nucleic acids in the biological sample during the preparation of a nucleic acid-containing sample to be supplied to the test from the biological sample so that the sample can be stably preserved until the time of operations of the test.

Regarding the method for stably preserving a biological sample prior to the nucleic acid extraction, for example, Patent Document 2 discloses a method in which a biological sample is exposed to a composition containing a chelating agent, such as sodium citrate, sodium borate, sodium fluoride, and EDTA, which is bindable to a trace amount of metal necessary for proteolytic activities of proteolytic substances and/or nucleolytic degradation activities of nucleolytic degradation substances in the sample. This method is to suppress the decomposition of nucleic acids during the preservation, by mixing the biological sample with the chelating agent-containing composition.

In addition, regarding the method for simultaneously conducting a stable storage and a pretreatment of DNA in a biological sample for use in PCR or the like, for example, Patent Document 3 discloses a method in which a biological sample is mixed with a storage reagent that comprises alkali and an organic solvent which is selected from the group consisting of a glycol, an alcohol, and a mixture thereof, and the mixture is stored at temperatures ranging from about -80°C to about 50°C. In this method, DNA in the biological sample is stabilized by glycol or alcohol, and at the same time DNA is released by lysing the biological sample through an alkali treatment. In other words, this method simultaneously conducts DNA preservation and DNA extraction from the biological sample.

On the other hand, in cases where nucleic acids are recovered from a biological sample, the carry-over of impurities (brought-in matter other than nucleic acids) which have been originally contained in the biological sample increases, and it is often difficult to recover nucleic acids with sufficient purity. When the purity of nucleic acids recovered from a biological sample is not enough, there is a problem in that the reliability is low in the results obtained from a test/analysis with use of the thus recovered nucleic acids, likewise of cases where the recovery efficiency is insufficient. Therefore, several methods for restraining the carry-over of inhibitory substances in a biological sample such as feces during the extraction/purification of nucleic acids from the biological sample, have been disclosed. For example, Patent Document 4 discloses a method for enzymatic amplification of a nucleic acid, including: eliminating substances that inhibit enzymatic amplification reactions of a nucleic acid by washing a test sample with an organic solvent; and enzymatically amplifying the nucleic acid of a cell contained in the test sample.

### References

### Patent Documents

Patent Document 1: Japanese Unexamined Patent Application, First Publication No. H 5-103672
Patent Document 2: Japanese Unexamined Patent Application, First Publication No. 2004-159648
Patent Document 3: Published Japanese Translation No. 2008-526226 of the PCT International Publication
Patent Document 4: PCT International Publication No. WO 00/08136 pamphlet

### SUMMARY OF THE INVENTION

In Examples described in Patent Document 2 mentioned above, urine which contains relatively little impurities is used as a biological sample, and it is shown that gonococcal DNA in urine can be stably preserved by the chelating agent-containing composition. However, when it comes to a biological sample which contains lots of impurities such as feces, it is difficult to sufficiently stabilize nucleic acids by such a composition merely using a chelating agent alone as an active ingredient.

Moreover, in the method described in Patent Document 3 mentioned above, preservation and nucleic acid extraction from a biological sample are simultaneously conducted with use of an alkali storage reagent, meaning that nucleic acids are preserved while being extracted in the storage reagent.
In other words, the nucleic acids are preserved in the alkali solution. Thus, there is a problem in that these nucleic acids are susceptible to decomposition during the preservation period.

On the other hand, in the method described in Patent Document 4 mentioned above, it is necessary to perform a solid-liquid separation process in which a biological sample such as feces is washed with an organic solvent, so as to restrain the carry-over of inhibitory substances. In the solid-liquid separation process, centrifugal separation, filtration with filters, and such treatments are usually performed. However, as the number of such treatment steps increases, the manipulation becomes complicated and furthermore contaminants derived from the biological sample and such matters may spread.

It is an object of the present invention to provide a method capable of detecting a nucleic acid from a biological sample such as feces with sufficient precision without a need for complicated manipulations.

### Means for Solving the Problems

The inventors of the present invention have conducted intensive studies to solve the above-mentioned problems. As a result, they discovered that a nucleic acid in a biological sample can be detected with sufficient precision not by performing extraction or purification of the nucleic acid, but by mixing a collected biological sample with a nucleic acid stabilizer solution comprising a water-soluble organic solvent as an active ingredient to prepare a suspension, and directly using this suspension or a suspension made by diluting this suspension in a reverse transcription reaction and/or a nucleic acid amplification reaction. This has led to the completion of the present invention.

That is, the present invention provides the following aspects.
(1) A method for detecting a nucleic acid in a biological sample, comprising:
   (A) mixing a biological sample with a nucleic acid stabilizer solution comprising a water-soluble organic solvent as an active ingredient, and then preserving the thus yielded suspension for a predetermined period of time; and
   (B) performing a reverse transcription reaction and/or a nucleic acid amplification reaction with use of a part of the suspension yielded in (A) as a template solution, thereby detecting a nucleic acid in the suspension.
(2) The method for detecting a nucleic acid in a biological sample according to the aspect (1), wherein the suspension yielded in (A) is diluted and then a part of the diluted suspension is used as a template solution in (B).
(3) The method for detecting a nucleic acid in a biological sample according to either one of the aspects (1) and (2), wherein the nucleic acid stabilizer solution includes one or more types selected from the group consisting of a water-soluble alcohol, a ketone, and an aldehyde, as the water-soluble organic solvent.
(4) The method for detecting a nucleic acid in a biological sample according to the aspect (3), wherein the water-soluble organic solvent includes one or more types selected from the group consisting of ethanol, propanol, and methanol, as the water-soluble alcohol.
(5) The method for detecting a nucleic acid in a biological sample according to the aspect (3), wherein the water-soluble organic solvent includes acetone and/or methyl ethyl ketone, as the ketone.
(6) The method for detecting a nucleic acid in a biological sample according to any one of the aspects (1) to (5), wherein the pH of the nucleic acid stabilizer solution is from 2 to 6.5.
(7) The method for detecting a nucleic acid in a biological sample according to any one of the aspects (1) to (6), wherein the period of time for preserving the suspension in (A) is one hour or longer.
(8) The method for detecting a nucleic acid in a biological sample according to any one of the aspects (2) to (7), wherein the suspension is diluted in (B)by adding any one or more types selected from the group consisting of an acidic buffer solution whose pH is 2 or higher, the nucleic acid stabilizer solution, and purified water.
(9) The method for detecting a nucleic acid in a biological sample according to the aspect (8), wherein the acidic buffer solution is a buffer solution selected from the group consisting of an acetic acid/sodium acetate buffer system, a citric acid/sodium hydroxide buffer system, and a lactic acid/sodium lactate buffer system.
(10) The method for detecting a nucleic acid in a biological sample according to any one of the aspects (1) to (9), wherein the nucleic acid stabilizer solution further includes one or more types selected from the group consisting of a protease inhibitor and a polycation.
(11) The method for detecting a nucleic acid in a biological sample according to any one of the aspects (1) to (10), wherein the biological sample is feces, blood, or urine.
(12) The method for detecting a nucleic acid in a biological sample according to any one of the aspects (1) to (11), wherein the method is to detect a nucleic acid derived from a mammalian cell contained in the biological sample.
(13) The method for detecting a nucleic acid in a biological sample according to the aspect (12), wherein the mammalian cell is a gastrointestinal tract cell or an exfoliated large intestine cell.
(14) The method for detecting a nucleic acid in a biological sample according to either one of the aspects (12) or (13), wherein the nucleic acid derived from a mammalian cell is a marker for neoplastic transformation or a marker for an inflammatory gastrointestinal tract disease.
(15) The method for detecting a nucleic acid in a biological sample according to either one of the aspects (12) or (13), wherein the nucleic acid derived from a mammalian cell is a nucleic acid derived from a *Cox-2* gene.

By the method for detecting a nucleic acid in a biological sample of the present invention, a nucleic acid in a biological sample can be detected with sufficient precision without performing extraction or purification of the nucleic acid from the biological sample. The method for detecting a nucleic acid in a biological sample of the present invention is particularly suitable for the detection of a nucleic acid in a biological sample such as feces in which relatively lots of impurities are contained and nucleic acids are easily damaged.

### BRIEF DESCRIPTION OF THE DRAWINGS

FIG. 1 is a graph showing assay results of the *MDR1* gene expression level in Example 1.
FIG 2 includes graphs showing calculation results of relative values of the *GAPDH* gene expression level in Example 2.
FIG 3 includes graphs showing calculation results of relative values of the *GAPDH* gene expression level in Reference Example 1.

### DETAILED DESCRIPTION OF THE INVENTION

In the present invention and the description of this application, the term "inhibitory substance" refers to a substance which inhibitorily acts on an enzymatic reaction where a nucleic acid is used as a substrate. The enzymatic reaction is not specifically limited as long as a nucleic acid is used as a substrate in the enzymatic reaction. For example, what can be enumerated are a reverse transcription reaction, a nucleic acid amplification reaction, and such enzymatic reactions that are generally used for nucleic acid analyses. Here, the term "nucleic acid amplification reaction" refers to a reaction in which a nucleic acid is amplified by utilizing a nucleotide chain elongation reaction with a polymerase or a ligase. Examples of the nucleotide chain elongation reaction with a polymerase can include PCR (polymerase chain reaction), real-time PCR, SDA (standard displacement amplification), and the like. Examples of the nucleotide chain elongation reaction with a ligase can include LCR (ligase chain reaction), and the like.
The inhibitory substance can be specifically exemplified by a bile acid, a bile salt, or the like.

The method for detecting a nucleic acid in a biological sample of the present invention (hereunder, may be referred to as the method for detecting a nucleic acid of the present invention") is a method for detecting a nucleic acid in a biological sample, comprising the following (A) and (B):
(A) mixing a biological sample with a nucleic acid stabilizer solution comprising a water-soluble organic solvent as an active ingredient, and thereafter preserving the thus yielded suspension for a predetermined period of time; and
(B) performing a reverse transcription reaction and/or a nucleic acid amplification reaction with use of a part of the suspension yielded from the step (A) as a template solution, to thereby detect a nucleic acid in the suspension.

The method for detecting a nucleic acid of the present invention is characterized in that a nucleic acid contained in a biological sample is used as a template of a reverse transcription reaction and/or a nucleic acid amplification reaction, not having been extracted nor purified, but in a state of being suspended in a nucleic acid stabilizer solution which comprises a water-soluble organic solvent as an active ingredient. In the reverse transcription reaction and/or the nucleic acid amplification reaction, if an insoluble matter (solid component) derived from a biological component is present in the reaction system, the reaction is inhibited, and it is difficult to have reliable results. Particularly in cases of a biological sample such as feces which contains lots of inhibitory substances, large amounts of inhibitory substances are to be carried over to the reaction system together with the insoluble matter. On the other hand, in the method for detecting a nucleic acid of the present invention, by preserving a suspension that comprises a mixture of a biological sample and a nucleic acid stabilizer solution having a water-soluble organic solvent as an active ingredient, for a predetermined period of time, and thereafter adding the suspension to a reaction solution, it becomes possible to satisfactorily obtain a reaction product as desired through a reverse transcription reaction and/or a nucleic acid amplification reaction, similarly to cases where a nucleic acid extracted/purified from a biological sample is used.

In the method for detecting a nucleic acid of the present invention, the reason why a nucleic acid in a biological sample can be detected with excellent precision through a reverse transcription reaction and/or a nucleic acid amplification reaction, without performing extraction or purification from the biological sample, is not clear. However, it can be attributed to a nucleic acid-stabilizing effect and an inhibitory substance-leaching effect of the water-soluble organic solvent. Because of the nucleic acid-stabilizing effect of the water-soluble organic solvent, even though the suspension comprising the nucleic acid stabilizer solution and the biological sample is preserved for a long period of time, nucleic acids in the suspension can be stably preserved. In addition, by immersing the biological sample in the nucleic acid stabilizer solution comprising the water-soluble organic solvent for a predetermined period of time, inhibitory substances can be leached from the solid component in the biological sample. Accordingly, by mixing the biological sample with a sufficient amount of the nucleic acid stabilizer solution, it becomes possible to prepare a suspension in which the amount of inhibitory substances contained in the solid component itself, where nucleic acids are present, has been reduced to a degree that would not influence the reverse transcription reaction or such reactions. By so doing, in cases where the suspension containing the solid component is added to the reaction solution, it is possible to prevent the carry-over of excessive inhibitory substances from the solid component, and to detect a stably preserved nucleic acid with excellent precision.

Hereunder is a description of the respective steps.
Firstly, as the step (A), a biological sample is mixed with a nucleic acid stabilizer solution comprising a water-soluble organic solvent as an active ingredient, and then the thus yielded suspension is preserved for a predetermined period of time. By mixing the biological sample with the nucleic acid stabilizer solution comprising a water-soluble organic solvent as an active ingredient, nucleic acids in the biological sample can be stably preserved. Since nucleic acids in a biological sample are previously stabilized in this way, a nucleic acid in the biological sample containing lots of impurities such as microorganisms and enzymes can be detected with excellent precision while minimizing a time-wise alteration of the nucleic acid in the biological sample in terms of the molecular profiling.

The reason why nucleic acids in a biological sample can be stably preserved by mixing the sample with a water-soluble organic solvent, is not clear. However, it can be attributed to a dewatering effect of the water-soluble organic solvent component, because the cellular activities of mammalian cells, microorganisms, and the like, contained in the biological sample are remarkably reduced; and it can also be attributed to a protein-denaturing effect of the water-soluble organic solvent component, because the activities of various kinds of degrading enzymes such as proteases, DNases, and RNases in the biological sample are remarkably reduced.

In the present invention and the description of this application, the term "water-soluble organic solvent" refers to an organic solvent that is highly soluble with water or mixable with water at any optional ratio. Biological samples such as feces usually have high moisture contents. For this reason, a biological sample and a nucleic acid stabilizer solution can be quickly mixed by using such a water-soluble organic solvent that is highly soluble with water or mixable with water at any optional ratio, as an active ingredient of the nucleic acid stabilizer solution.

The water-soluble organic solvent to be used as an active ingredient of the nucleic acid stabilizer solution specifically refers to a solvent which is a kind of alcohol, ketone, or aldehyde, of a straight chain structure, and which is in a liquid state around room temperature, for example, from 15 to 40°C. By having such a water-soluble organic solvent of a straight chain structure as an active ingredient rather than having an organic solvent of a cyclic structure, such as a benzene ring, as an active ingredient, the nucleic acid stabilizer solution can be quickly mixed with a biological sample. In general, organic solvents of a cyclic structure are prone to separate from water. Thus, this kind of solvent can be hardly mixed with a biological sample such as feces, and it is difficult to achieve a high nucleic acid-stabilizing effect. This is because that, even using a solvent that is reasonably soluble with water, it is often necessary to vigorously mix or heat it so as to homogeneously disperse a biological sample such as feces therein. In order to facilitate the mixing between a biological sample and an organic solvent of a cyclic structure, it can also be considered to prepare a mixture solution of the organic solvent and water in advance, and then to mix the biological sample and the mixture solution. However, this technique is not preferable because it is often necessary to vigorously mix or heat the organic solvent of a cyclic structure and water so as to prepare the mixture solution.

It is preferable that the active ingredient of the nucleic acid stabilizer solution is a water-soluble organic solvent having a water solubility of 12% by weight or higher, more preferably a water-soluble organic solvent having a water solubility of 20% by weight or higher, still more preferably a water-soluble organic solvent having a water solubility of 90% by weight or higher, and particularly preferably a water-soluble organic solvent that is mixable with water at any optional ratio. The water-soluble organic solvent that is mixable with water at any optional ratio can be exemplified by methanol, ethanol, n-propanol, 2-propanol, acetone, and formaldehyde.

The water-soluble organic solvent for use as an active ingredient of the nucleic acid stabilizer solution is not particularly limited as long as the solvent fulfills the above-mentioned definition and is capable of exerting a high effect to recover nucleic acids. Examples of such a water-soluble organic solvent can include: when it comes to alcohols, methanol, ethanol, propanol, butanol, mercaptoethanol, and the like, being water-soluble alcohols; when it comes to ketones, acetone, methyl ethyl ketone (having a water solubility of 90% by weight), and the like; and, when it comes to aldehydes, acetaldehyde (acetyl aldehyde), formaldehyde (formalin), glutaraldehyde, paraformaldehyde, glyoxal, and the like. The propanol may be either n-propanol or 2-propanol. Moreover, the butanol may be either 1-butanol (having a water solubility of 20% by weight) or 2-butanol (having a water solubility of 12.5% by weight). The water-soluble organic solvent for use in the present invention is preferably a water-soluble alcohol, acetone, methyl ethyl ketone, or formaldehyde. This is because that these solvents have sufficiently high water solubility. In terms of availability, ease of handling, and safety, more preferred are water-soluble alcohols, and much more preferred are ethanol, propanol, and methanol. In particular, ethanol is the safest and can be easily handled in domestic households. Therefore, ethanol is particularly useful in screening tests for routine checkups or the like.

The concentration of the water-soluble organic solvent in the nucleic acid stabilizer solution is not particularly limited as long as the nucleic acid-stabilizing effect can be exerted with this concentration. The concentration can be appropriately determined by considering the type of the water-soluble organic solvent and the like. By having a sufficiently high concentration of the water-soluble organic solvent in the nucleic acid stabilizer solution, it becomes possible, when mixing a biological sample and the nucleic acid stabilizer solution, to quickly exert the above-mentioned effect because the water-soluble organic solvent component is rapidly infiltrated into the whole biological sample.

For example, when a water-soluble alcohol or a ketone is used, the concentration of the water-soluble organic solvent in the nucleic acid stabilizer solution is preferably 30% or higher, more preferably 50% or higher, still more preferably from 50 to 80%, and particularly preferably from 60 to 70%. With higher concentration of the water-soluble organic solvent, more sufficient effect can be achieved by using even a small amount of the nucleic acid stabilizer solution relative to feces having high moisture content.

Moreover, when acetone or methyl ethyl ketone is used, the concentration of the water-soluble organic solvent in the nucleic acid stabilizer solution is preferably 30% or higher, more preferably 60% or higher, and still more preferably 80% or higher. Alternatively, when acetaldehyde, formaldehyde, glutaraldehyde, paraformaldehyde, or glyoxal is used as an active ingredient, the concentration of the water-soluble organic solvent in the nucleic acid stabilizer solution is preferably from 0.01% to 30%, more preferably from 0.03% to 10%, and still more preferably from 3% to 5%. Aldehydes are more capable of exerting effects even at low concentrations than alcohols and ketones.

In addition, the nucleic acid stabilizer solution to be used in the present invention may contain either only a single type of water-soluble organic solvent or a mixture solution of two or more types of water-soluble organic solvents. For example, the water-soluble organic solvent may be a mixture solution of two or more types of alcohols, or a mixture solution of an alcohol and another type of water-soluble organic solvent. It is also preferable that the water-soluble organic solvent be a mixture solution of an alcohol and acetone because the nucleic acid-stabilizing effect is much improved.

The nucleic acid stabilizer solution for use in the present invention can be adjusted to a desired concentration by diluting the water-soluble organic solvent with an appropriate solvent. The solvent for use in the dilution of the water-soluble organic solvent is not particularly limited as long as the effects of the water-soluble organic solvent are not impaired. For example, water, buffer solution such as PBS, or the like may be employed.

The nucleic acid stabilizer solution for use in the present invention is preferably a solution in which the water-soluble organic solvent is diluted with a buffer solution whose pH is from 2 to 7.5, and more preferably a solution in which the water-soluble organic solvent is diluted with an acidic buffer solution having a buffering effect to maintain the pH within a range of 2 or higher. The pH of this acidic buffer solution is preferably from 2 to 6.5, more preferably from 3 to 6, still more preferably from 3.5 to 5.5, and particularly preferably from 4.0 to 5.0. By retaining a biological sample in an acidic condition, hydrolysis of nucleic acids in the biological sample can be more effectively suppressed.

As the acidic buffer solution for use in the dilution of the water-soluble organic solvent for preparing the nucleic acid stabilizer solution, preferred is a buffer solution which contains an organic acid and a conjugate base of the organic acid to exert a buffering effect by using the organic acid and the conjugate base thereof. Particularly preferred is a buffer solution selected from the group consisting of a citric acid/sodium hydroxide buffer system, a lactic acid/sodium lactate buffer system, and an acetic acid/sodium acetate buffer system. This kind of buffer solution can be prepared, for example, by adding an organic acid and either an alkali metal salt or an alkali earth metal salt of the organic acid to water or to an appropriate solvent, thereby adjusting the pH as desired. Alternatively, it is also possible to add an organic acid to water or to an appropriate solvent, and thereafter use a hydroxide of an alkali metal or an alkali earth metal, thereby adjusting the pH as desired.

Alternatively, as the acidic buffer solution for use in the dilution of the water-soluble organic solvent, it is also possible to use a solution which contains both an organic acid and a mineral acid to have an appropriate buffering effect. For example, a glycine/HCl buffer system, a sodium cacodylate/HCl buffer system, a phthalic acid HK/HCl buffer system, and such a buffer system which has a buffering effect on the acid side may be employed.

In the present invention and the description of this application, the pH of the acidic buffer solution means a value obtained by measurement with a pH meter which adopts the glass electrode method as the principal of measurement (for example, a product of DKK-TOA Corporation) after calibration with a phthalate reference solution and a neutral phosphate reference solution.

The nucleic acid stabilizer solution for use in the present invention can be added with other ingredients as long as the nucleic acid-stabilizing effect of the water-soluble organic solvent is not impaired. Such ingredients can be exemplified by a protease inhibitor, a polycation, a salt, and the like.

Usually, nucleic acids in a biological sample are present while being included inside a cell. Then, proteins and such constituents of the cell membrane are decomposed by proteases in the biological sample. As a result, the nucleic acids flow out to the outside the cell through pores or the like generated in the cell membrane, and these nucleic acids and such cell-derived components that have flown out to the outside the cell are also decomposed by the action of nucleic acid degrading enzymes and the like which are abundantly present in the biological sample. Here, by using a protease inhibitor as an active ingredient of the nucleic acid stabilizer solution, the decomposition of cell membrane proteins in a biological sample can be effectively suppressed, and nucleic acids and such cell-derived components can be kept inside the cell where degrading enzymes are in comparatively low abundance. By so doing, the preservability of the cell-derived components can be improved.

The protease inhibitor to be added to the nucleic acid stabilizer solution is not particularly limited as long as it is capable of inhibiting an enzymatic activity of a protease (an enzyme capable of catalyzing hydrolysis of a peptide bond). A proteinase inhibitor or a peptidase inhibitor can also be employed. In addition, it is possible to use a substance capable of inhibiting a serine protease, a substance capable of inhibiting a cysteine protease, a substance capable of inhibiting an aspartic protease (acidic protease), or a substance capable of inhibiting a metallo protease.

The protease inhibitor to be added to the nucleic acid stabilizer solution can be appropriately selected from known protease inhibitors. The protease inhibitor for use in the present invention can be exemplified by AEBSF, aprotinin, bestain, calpain inhibitor I, calpain inhibitor II, chymostatin, 3,4-dichloroisocoumain, E-64, lactacystin, leupeptin, MG-115, MG-132, pepstatin A, PMSF, a proteasome inhibitor, TLCK, TPCK, a trypsin inhibitor, and the like. Alternatively, an ensemble of a plurality of types of protease inhibitors which is generally called a "protease inhibitor cocktail" can also be used.

In addition, the concentration of the above-mentioned protease inhibitor to be added to the nucleic acid stabilizer solution is not particularly limited as long as the concentration is enough to inhibit a protease in a biological sample. The concentration can be appropriately determined by considering the type of the protease inhibitor to be added, the blending ratio of the protease inhibitor relative to the biological sample, the pH and the temperature of the suspension prepared by mixing the nucleic acid stabilizer solution with the biological sample, and the like. Table 1 shows preferable concentrations of respective protease inhibitors in a suspension prepared by mixing a nucleic acid stabilizer solution with a biological sample.

**[Table1]**

| Protease inhibitor | Concentration |
|---|---|
| AEBSF | 0.1 to 1.0 mg/ml |
| Aprotinin | 0.06 to 2 µg/ml |
| Bestain | 4 to 400 µg/ml |
| Calpain Inhibitor I | 1 to 100 µg/ml |
| Calpain Inhibitor II | 1 to 100 µg/ml |
| Chymostatin | 6 to 60 µg/ml |
| 3,4-Dichloroisocoumain | 1 to 43 µg/ml |
| E-64 | 0.5 to 10 µg/ml |
| Lactacystin | 1 to 100 µM |
| Leupeptin | 0.1 to 10 µg/ml |
| MG-115 | 0.1 to 10 µM |
| MG-132 | 0.1 to 10 µM |
| Pepstatin A | 0.7 µg/ml |
| PMSF | 17 to 170 µg/ml |
| Proteasome Inhibitor | 0.1 to 10 µM |
| TLCK | 37 to 50 µg/ml |
| TPCK | 70 to 100 µg/ml |
| Trypsin Inhibitor | 10 to 100 µg/ml |

The protease inhibitor to be added to the nucleic acid stabilizer solution may be a peptide-based protease inhibitor as described above, or a reductant, a protein denaturant, or a chelating agent. Note that, in the present invention and the description of this application, the term "peptide-based protease inhibitor" refers to a peptide that is capable of inhibiting the activity of a protease through interaction with a protease, or a modified body thereof.

The chelating agent can be exemplified by ethylene diamine tetraacetic acid (EDTA), O,O'-bis(2-aminophenyl)ethyleneglycol-N,N,N',N'-tetraacetic acid (BAPTA), N,N-bis(2-hydroxyethyl)glycine (Bicine), trans-l,2-diaminocyclohexane-N,N,N',N'-tetraacetic acid (CyDTA), 1,3-diamino-2-hydroxypropane tetraacetic acid (DPTA-OH), diethylene triamine pentaacetic acid (DTPA), ethylenediamine-N,N'-dipropionic acid dihydrochloride (EDDP), ethylenediamine di(methylenephosphonic acid) hydrate (EDDPO), N-(2-hydroxyethyl)ethylenediamine triacetic acid (EDTA-OH), ethylenediamine tetra(methylenephosphonic acid) (EDTPO), O,O'-bis(2-aminoethyl)ethylene glycol tetraacetic acid (EGTA), N,N-bis(2-hydroxybenzyl)ethylenediamine diacetic acid (HBED), hexamethylene 1,6-diamine N,N,N',N'-tetraacetic acid (HDTA), N-(2-hydroxyethyl)iminodiacetic acid (HIDA), iminodiacetic acid (IDA), 1,2-diaminopropane tetraacetic acid (Methyl-EDTA), nitrilotriacetic acid (NTA), nitrilotripropionic acid (NTP), nitrilotri(methylphosphonic acid) trisodium salt (NTPO), tetrakis(2-pyridylmethyl)ethylenediamine (TPEN), triethyleneteraaminehexaacetic acid (TTHA), or the like.
The concentration of the chelating agent to be added as a protease inhibitor to a biological sample is not particularly limited as long as the concentration is enough to inhibit a protease in the biological sample. The concentration can be appropriately determined by considering the type of the chelating agent and the like. It is preferable to add the respective chelating agent so that the final concentration thereof in the suspension prepared by mixing the nucleic acid stabilizer solution with the biological sample be from 0.1 mM to 1 M.

The reductant can be exemplified by DTT (dithiothreitol), β mercaptoethanol, or the like.
The concentration of the reductant to be added as a protease inhibitor to a biological sample is not particularly limited as long as the concentration is enough to inhibit a protease in the biological sample. The concentration can be appropriately determined by considering the type of the reductant and the like. It is preferable to add the respective reductant so that the final concentration thereof in the suspension prepared by mixing the nucleic acid stabilizer solution with the biological sample be from 0.1 mM to 1M.

The protein denaturant can be exemplified by urea, guanine, a guanidine salt, or the like.
The concentration of the protein denaturant to be added as a protease inhibitor to a biological sample is not particularly limited as long as the concentration is enough to inhibit a protease in the biological sample. The concentration can be appropriately determined by considering the type of the protein denaturant and the like. It is preferable to add the respective protein denaturant so that the final concentration thereof in the suspension prepared by mixing the nucleic acid stabilizer solution with the biological sample be from 0.1 mM to 10 M.

The nucleic acid stabilizer solution may contain either only a single type of protease inhibitor or two or more types of protease inhibitors, in addition to the water-soluble organic solvent. Moreover, it is also possible to use a combination of a plurality of types of peptide-based protease inhibitors such as AEBSF, or to use different types of protease inhibitors that can be exemplified by a peptide-based protease inhibitor and a chelating agent, or a peptide-based protease inhibitor and a reductant.

In the present invention, it is also preferable to use a polycation as an active ingredient of the nucleic acid stabilizer solution, in addition to the water-soluble organic solvent. It becomes possible, by mixing a biological sample with a polycation, to efficiently reduce decomposition/synthesis reactions of nucleic acids caused by impurities contained in the biological sample. In addition, although lots of inhibitory substances are contained in a biological sample, it becomes possible, by mixing a biological sample with a polycation, to reduce inhibitory actions caused by these inhibitory substances.

In the present invention and the description of this application, the term "polycation" refers to a polymer compound that has a repeated structure including cationic functional groups, or a salt thereof. The cation can be exemplified by an amino group or the like. Specific examples of such a polycation include polypeptides such as polylysine as shown in the following formula (1) in which cationic functional groups are held on side chains, and polymers such as polyacrylamide produced by polymerizing monomers in which cationic functional groups are held on side chains. These
polypeptides and polymers suffice if the molecule is electrically cationic as a whole, and it is not necessary to have cationic functional groups on side chains of all repeating units (amino acid or monomer), although it is preferable to have cationic functional groups on side chains of all repeating units. In addition to these polylysine and polyacrylamide, other specific examples of such a polycation include polyvinylamine, polyallylamine, polyethylamine, polymethallylamine, polyvinylmethylimidazole, polyvinylpyridine, polyarginine, chitosan, 1,5-dimethyl-1,5-diazaundecamethylene-polymethobromide, poly(2-dimethylaminoethyl(meth) acrylate), poly(2-diethylaminoethyl(meth) acrylate), poly(2-trimethylammoniumethyl(meth) acrylate), polydimethylaminomethylstyrene, polytrimethylammoniummethylstyrene, polyornithine, and polyhistidine. In the present invention, it is preferable that the polycation is either polylysine or polyacrylamide, and it is more preferable that the polycation is polylysine. When a polycation is added to the nucleic acid stabilizer solution, it is either possible to use a single type of polycation or to use two or more types of polycations.

[Formula 1]

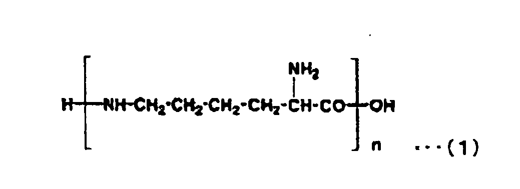

The concentration of the polycation to be added to a biological sample is not particularly limited as long as the concentration is enough to produce an effect to weaken the inhibitory action of an inhibitory substance contained in a biological sample (inhibitory action-weakening effect). The concentration can be appropriately determined by considering the type of the polycation, the type of the biological sample, the pH of the nucleic acid stabilizer solution, the blending ratio of the nucleic acid stabilizer solution to the biological sample, and the like. For example, in cases where polylysine is added as a polycation, it is preferable that the polylysine concentration in the nucleic acid stabilizer solution be from 0.01 to 1.0 m % by weight, more preferably from 0.0125 to 0.8 m % by weight, and still more preferably from 0.05 to 0.4 m % by weight. Note that, in the description of this application, the term "m % by weight" refers to "× 10⁻³ % by weight".

Furthermore, a high salt concentration solution may also be used as a nucleic acid stabilizer solution. The reason why a high salt concentration solution is suitable as a nucleic acid stabilizer solution is not clear. However, it can be considered such that various kinds of degrading enzymes are deposited through salting out, and thereby, due to the dewatering effect of the high concentration of salt component, the cellular activities of mammalian cells and bacteria such as resident microbes in the intestines are so remarkably reduced that the time-wise alteration is suppressed, and also, the environment is so out of the optimum salt concentration that the activities of various kinds of degrading enzymes such as proteases, DNases, and Rnases, in feces are remarkably reduced.

The salt to be contained as an active ingredient in the nucleic acid stabilizer solution can be appropriately selected from usual salts for use in the preparation or analysis of a biological sample. For example, the salt may be a hydrochloride, a sulfate, or an acetate. In addition, as an active ingredient, it is either possible to use a single type of salt, or to use a combination of two or more types of salts. It is preferable that the nucleic acid stabilizer solution for use in the present invention contains, as an active ingredient, one or more types of salts selected from the group consisting of sodium chloride, potassium chloride, ammonium sulfate, heavy ammonium sulfate, ammonium chloride, ammonium acetate, cesium sulfate, cadmium sulfate, cesium iron (II) sulfate, chromium (III) sulfate, cobalt (II) sulfate, copper (II) sulfate, lithium chloride, lithium acetate, lithium sulfate, magnesium sulfate, manganese sulfate, sodium sulfate, sodium acetate, sodium sulfate, zinc chloride, zinc acetate, and zinc sulfate. Of these, in terms of availability, ease of handling, and safety, it is preferable to use a solution containing sodium chloride and/or ammonium sulfate. In particular, sodium chloride is the safest and can be easily handled in domestic households. Therefore, sodium chloride is particularly useful in screening tests for routine checkups or the like.

The concentration of the salt contained as an active ingredient in the nucleic acid stabilizer solution (hereunder, may be simply referred to as "salt concentration") is not particularly limited as long as the function as a nucleic acid stabilizer can be achieved with this concentration. The concentration can be appropriately determined by considering the types of salt and solvent to be used, and the like. The upper limit of salt concentration is the saturating concentration of the respective salt. On the other hand, the lower limit varies depending on the type of salt to be used, although it is possible for those skilled in the art to obtain the lower limit through preliminary experiments.

For example, the lower limit can be obtained by the following manner. First, salt solutions of a plurality of concentrations below the saturating concentration are prepared. Nucleic acids are recovered from feces that have been immersed in these salt solutions for a predetermined period of time.
The minimum value of salt concentration at which the amount of the thus recovered nucleic acids exceeds the amount of nucleic acids recovered from feces which are not treated with any salt solution, can be deemed as the lower limit of salt concentration of salt to be contained as an active ingredient. Moreover, for example, in cases where feces are used as a biological sample, a bacterial culture solution, or a mixture solution including a bacterial culture solution and a culture solution of a mammalian cell line, can be used as an artificial fecal sample substituting for feces.

In the present invention, in order to achieve a much higher nucleic acid-stabilizing effect, it is preferable that the salt concentration of the nucleic acid stabilizer solution is 1/2 or higher concentration of the saturating concentration of salt serving as an active ingredient, regardless of the type of salt, more preferably 4/5 or higher concentration of the saturating concentration, still more preferably in a vicinity of the saturating concentration, and particularly preferably substantially the saturating concentration. By having a sufficiently high salt concentration, it becomes possible, when mixing feces with a nucleic acid stabilizer solution of the high salt concentration, to quickly stabilize nucleic acids because the component is rapidly infiltrated into the feces. In addition, with higher salt concentration, sufficient effects can be exerted even if the amount of the nucleic acid stabilizer solution is small relative to feces having high moisture content. The "solution having 1/2 or higher concentration of the saturating concentration" and the "solution having 4/5 or higher concentration of the saturating concentration" can be prepared for example by appropriately diluting a saturated solution that has been prepared by a usual method, with a solvent.

For example, when sodium chloride is used as an active ingredient, the salt concentration is preferably 13% (wt/wt) or higher, more preferably 20% (wt/wt) or higher, still more preferably 26% (wt/wt) or higher, and particularly preferably from 26% (wt/wt) to the saturating concentration. When ammonium sulfate is used, the salt concentration is preferably 20% (wt/wt) or higher, more preferably 30% (wt/wt) or higher, and still more preferably from 30 to 46% (wt/wt).

The nucleic acid stabilizer solution to be used in the present invention can be added with other substances such as a chaotropic salt, a surfactant, and a colorant, as long as the nucleic acid-stabilizing effect of the water-soluble organic solvent is not impaired.

By adding a chaotropic salt or a surfactant to the nucleic acid stabilizer solution, the cellular activities in the biological sample or the enzymatic activities of various kinds of degrading enzymes can be more effectively inhibited. The chaotropic salt to be added to the nucleic acid stabilizer solution can be exemplified by guanidine hydrochloride, guanidine isothiocyanate, sodium iodide, sodium perchlorate, sodium trichloroacetate, or the like. It is preferable that the surfactant to be added to the nucleic acid stabilizer solution is a nonionic surfactant. The nonionic surfactant can be exemplified by Tween 80, CHAPS (3-[3-cholamidopropyl dimethylammonio]-1-propanesulfonate), Triton X-100, Tween 20, or the like. The concentration of the chaotropic salt or the surfactant is not particularly limited as long as the nucleic acid-stabilizing effect can be achieved with this concentration. The concentration can be appropriately determined by considering the amount of the biological sample, the methods for recovering and analyzing a nucleic acid afterwards, and the like.

By adding a colorant to the nucleic acid stabilizer solution or to the suspension made by mixing the biological sample and the nucleic acid stabilizer solution, effects such as the prevention against accidental swallowing and the lightening of the color of the biological sample can be given. It is preferable that the colorant is a coloring agent for use as a food additive that exhibits blue, green, or such a color. Examples thereof can include Fast Green FCF (Green No. 3), Brilliant Blue FCF (Blue No. 1), and Indigo Carmine (Blue No. 2). Moreover, it is either possible to add a plurality of types of colorants in combination, or to add a single type of colorant alone.

The biological sample to be supplied to the method for detecting a nucleic acid of the present invention is not particularly limited as long as the biological sample includes cells, tissue pieces, or such insoluble matter (solid components). Specific examples thereof can include feces, urine, blood, bone marrow fluid, lymph fluid, sputum, saliva, semen, bile, pancreatic juice, ascites, exudate, amniotic fluid, intestinal lavage fluid, lung lavage fluid, bronchial lavage fluid, and bladder lavage fluid. Alternatively, the biological sample may be a culture product such as cultured cells. It is particularly preferable that the biological sample to be supplied to the method for detecting a nucleic acid of the present invention is feces, blood, urine, or a culture product such as cultured cells. In addition, the biological sample is not particularly limited as long as it is collected from a biological organism, although preferred is a sample derived from a mammal, and more preferably from a human. For example, it is preferable that the biological sample is collected from a human for the purpose of routine checkups, diagnosis, or such an occasion, although the biological sample may be from a domestic animal, a wild animal, or the like. Moreover, it is also possible to use a biological sample that has been preserved for a certain period of time after the collection, although it is preferable to use the sample right after the collection. In cases where the biological sample is feces, it is preferable that the feces are supplied to the method for detecting a nucleic acid of the present invention right after the excretion, although it is possible to supply it after a certain period of time after the excretion.

The amount of the biological sample to be supplied to the method for detecting a nucleic acid of the present invention is not particularly limited, and the amount can be appropriately determined by considering the type of the biological sample, the type of the reaction employed for the detection of the nucleic acid, and the like. For example, in cases of feces, the amount is preferably from 10 mg to 1 g. If the amount of feces is too large, the collection task becomes so bothersome and the size of the container for the collection of feces becomes so large that the handling property and such properties may be worsened. Conversely, if the amount of feces is too small, the number of mammalian cells, such as exfoliated large intestine cells, contained in the feces is so small that necessary amounts of nucleic acids cannot be recovered, and thus the precision of the analysis of the target nucleic acid may be worsened. In addition, because feces are heterogeneous, in other words, because diverse and various kinds of components are unevenly present in feces, it is preferable to collect a sample from wide area on feces at the time of collection, so as to avoid the influence of localization of mammalian cells.

In cases of a biological sample such as feces which contains lots of solid component, the volume of the nucleic acid stabilizer solution to be mixed with the collected biological sample is not particularly limited, but preferred blending ratio of the nucleic acid stabilizer solution to the biological sample is that the volume of the nucleic acid stabilizer solution relative to the volume of the biological sample is one or higher. This is because that, by using the nucleic acid stabilizer solution at an equal or larger volume of the biological sample, the entire surface of the biological sample can be immersed in the nucleic acid stabilizer solution, making it possible for the active ingredient such as a water-soluble organic solvent to sufficiently act on the biological sample. In particular, it becomes possible, by mixing the biological sample with five times or more volume of the nucleic acid stabilizer solution, to quickly and effectively disperse the biological sample in the nucleic acid stabilizer solution, and furthermore to reduce the influence of weakened concentration of the water-soluble organic solvent due to the moisture contained in the biological sample. On the other hand, it is often preferable that the total volume of the mixture of the biological sample and the nucleic acid stabilizer solution is relatively small, because the handling is easy. For example, in cases where feces are used, it is possible to collect feces in a collection container where a nucleic acid stabilizer solution has been previously provided, and then prepare the mixture in the container. In this case, for example, if the volumes of feces and the nucleic acid stabilizer solution are equal, it is possible to reduce the weight and the size of the collection container including the nucleic acid stabilizer solution. In this way, it becomes possible to improve both the property to handle the biological sample and the suspension to be obtained, and the dispersibility of the biological sample in the nucleic acid stabilizer solution, with a good balance. Thus, in cases where the biological sample is feces, it is preferable that the blending ratio of the biological sample to the nucleic acid stabilizer solution is from 1:1 to 1:20, more preferably from 1:3 to 1:10, and still more preferably about 1:5.

The mixing of the biological sample and the nucleic acid stabilizer solution in the step (A) may be conducted by immersing the biological sample in the nucleic acid stabilizer solution without performing any specific agitation operation. The reason is that, because the nucleic acid stabilizer solution for use in the present invention is highly amenable to even a biological sample such as feces which has high moisture content, it is possible, depending on the amount and the state of the biological sample to be mixed, to rapidly infiltrate the nucleic acid stabilizer solution into the biological sample and to exert a sufficient level of the nucleic acid-stabilizing effect only by merely immersing the biological sample in the nucleic acid stabilizer solution without performing any specific agitation operation.

The mixing of the biological sample and the nucleic acid stabilizer solution may also be conducted by charging the biological sample into the nucleic acid stabilizer solution to effect immersion, and thereafter agitating them. It becomes possible to sufficiently disperse and suspend the biological sample in the nucleic acid stabilizer solution by agitating them. In cases where the suspension is prepared by agitating the nucleic acid stabilizer solution having the biological sample charged therein, it is preferable to quickly conduct the agitation. The reason is that, by quickly dispersing the biological sample in the nucleic acid stabilizer solution, the active ingredient such as the water-soluble organic solvent can be rapidly infiltrated into cells in the biological sample, so that the water-soluble organic solvent and the like can rapidly act on impurities in the biological sample, making it possible to achieve a better nucleic acid-stabilizing effect.

The method of mixing the biological sample and the nucleic acid stabilizer solution to prepare the suspension is not particularly limited as long as the mixing is conducted by means of a physical technique. For example, the mixing may be conducted by placing a collected biological sample in a sealable container where the nucleic acid stabilizer solution has been charged in advance, sealing the container, and then inverting the container or shaking the container with use of a shaker such as a vortex mixer. Moreover, the biological sample and the nucleic acid stabilizer solution may be mixed in the presence of mixer particles. It is preferable to adopt a mixing method that uses a shaker or mixer particles because the mixing can be quickly conducted. In particular, by using a collection container in which mixer particles have been contained in advance, the mixing can be quickly conducted even in domestic households or such environments having no special equipment.

The mixer particles are not particularly limited as long as the particles are formed of a composition that does not impair the nucleic acid-stabilizing effect of the nucleic acid stabilizer solution, as well as having enough durability and specific gravity to quickly disperse a biological sample such as feces in a nucleic acid stabilizer solution by collision with the biological sample. The particles may be composed of either one type of material, or two or more types of materials. Examples of such mixer particles include particles composed of glass, ceramics, plastics, latex, metals, and the like. In addition, the mixer particles may be either magnetic or nonmagnetic.

In the step (A), the suspension made by mixing the biological sample and the nucleic acid stabilizer solution is preserved for a predetermined period of time. It becomes possible, by preserving the suspension, to sufficiently expose the biological sample to the water-soluble organic solvent so as to achieve a high nucleic acid-stabilizing effect. At the same time, it also becomes possible, by preserving the biological sample in a state of being mixed with the nucleic acid stabilizer solution for a predetermined period of time, to efficiently leach inhibitory substances contained in the biological sample into the nucleic acid stabilizer solution.

Such an inhibitory substance-leaching effect of the water-soluble organic solvent can be attributed to the difference between inhibitory substances and nucleic acids in terms of the solubility with the water-soluble organic solvent. In other words, inhibitory substances such as bile acid are easily dissolved with a water-soluble organic solvent such as alcohol whereas these are hardly dissolved with a nonpolar organic solvent. On the other hand, nucleic acids are hardly dissolved with a water-soluble organic solvent such as alcohol, because they are insolubilized due to the effect of salt in alcohol. Accordingly, it is considered such that, when a biological sample such as feces is mixed with a water-soluble organic solvent such as alcohol, inhibitory substances can be leached out into the water-soluble organic solvent, enabling separation from nucleic acids that are insoluble with the water-soluble organic solvent component.

The time for preserving the suspension is not particularly limited as long as the nucleic acid-stabilizing effect and the inhibitory substance-leaching effect can be exerted, and the time can be appropriately determined by considering the type and the concentration of the water-soluble organic solvent, the blending ratio of the biological sample to the nucleic acid stabilizer solution, the temperature of preservation, and the like. In the method for detecting a nucleic acid of the present invention, it is preferable that the preservation is conducted for one hour or longer, more preferably 12 hours or longer, still more preferably 24 hours or longer, and particularly preferably 72 hours or longer. In addition, the preservation may also be conducted for 168 hours or longer.

The nucleic acid-stabilizing effect and the inhibitory substance-leaching effect of the water-soluble organic solvent are not to be particularly influenced by a temperature condition so long as a sufficient amount of water-soluble organic solvent is present in the suspension. Accordingly, the method for detecting a nucleic acid of the present invention is able to suppress the loss of nucleic acids in a biological sample even at temperatures at which usually a biological sample such as feces is collected, that is to say, at room temperature. Note that, the suspension is preferably preserved at 50°C or lower temperature. This is because that, when preserved under a high temperature condition for a long period of time, the concentration of the water-soluble organic solvent in the mixture may decrease, due to volatilization or such an event, to a level lower than enough concentration to exert these effects.

The inhibitory substance-leaching effect of the water-soluble organic solvent is better exerted at higher temperatures for preserving the biological sample rather than low temperatures. Specifically speaking, in the present invention, the temperature for preserving the suspension in the step (A) is preferably 4°C or higher, and more preferably 20°C or higher.

In the method for detecting a nucleic acid of the present invention, the nucleic acid-stabilizing effect and the inhibitory substance-leaching effect can be exerted as long as the temperature for preserving the suspension in the step (A) is 4°C or higher. That is to say, it is either possible to preserve the suspension under a temperature-controlled environment with use of a thermostat or the like, or at room temperature without needing a special temperature-controlled environment. Moreover, the preservation can also be conducted at temperatures at which usually feces are collected, at which usually a fecal sample is transferred, or such situations. Accordingly, for example, in cases where the suspension prepared in the step (A) is to be transferred without any temperature control, this period of time for the transfer can be counted as the preservation period. More specifically, in cases where the place for preparing a fecal sample by the collector and the place for conducting the operations to detect a nucleic acid, are apart from each other, and the thus the prepared suspension has to be transferred to the place for conducting the operations to detect the nucleic acid, as with cases of routine checkups or the like; then, this period of time for the transfer can be counted as the preservation period in the step (A) as long as the temperature for the transfer is from 4 to 50°C, no matter whether the temperature is controlled or not.

Next, as the step (B), a reverse transcription reaction and/or a nucleic acid amplification reaction is/are performed with use of a part of the suspension yielded from the step (A) as a template solution, to thereby detect a nucleic acid in the suspension. At the time when the suspension is added to the reaction solution of the reverse transcription reaction or the like, the suspension is added to the reaction solution while being in a suspended state, in other words, insoluble matter (solid components) in the suspension are added to the reaction solution. Although the majority of nucleic acids in a biological sample are present inside solid components such as cells from the beginning, any operation to forcefully elute nucleic acids from the solid components is not performed in the present invention. For this reason, it is suggested that the majority of nucleic acids are present inside the solid components or in a state of being adhered to the surfaces of these solid components. Thus, the nucleic acids can be efficiently added to the reaction solution by adding the solid components in the suspension to the reaction solution.

In the preparation of the suspension in the step (A), depending on the blending ratio of the biological sample to the nucleic acid stabilizer solution, the concentration of inhibitory substances in the prepared suspension may be so high that the carry-over of inhibitory substances may influence the reverse transcription reaction and/or the nucleic acid amplification reaction, if the suspension is directly added to the reaction solution. In addition, depending on the composition of the nucleic acid stabilizer solution, the carry-over of the active ingredient itself of the nucleic acid stabilizer solution may influence the reaction, if the suspension is directly added to the reaction solution. In these cases, it is possible to dilute the suspension yielded from the step (A), and then to use a part of the diluted suspension as a template solution.

The dilution factor of the suspension is not particularly limited as long as the dilution factor is within a range capable of reducing the concentration of inhibitory substances in the diluted suspension to an extent such that the carry-over of inhibitory substances would not influence the reaction when added to the reaction solution of the reverse transcription reaction or the nucleic acid amplification reaction. The dilution factor can be appropriately determined by considering the type of the biological sample, the content of the inhibitory substances, the type of the water-soluble organic solvent, the blending ratio of the biological sample to the nucleic acid stabilizer solution, and the like.

The solvent to be added to the suspension for the purpose of dilution is not particularly limited as long as the nucleic acid-stabilizing effect and the inhibitory substance-leaching effect of the water-soluble organic solvent are not impaired. Specific examples thereof can include the solvents that have been enumerated as solvents for use in the dilution of the water-soluble organic solvent in the preparation of the nucleic acid stabilizer solution. Moreover, the nucleic acid stabilizer solution may also be used. In the present invention, it is preferable to dilute the suspension by adding an acidic buffer solution whose pH is 2 or higher, a nucleic acid stabilizer solution, or a purified water.

It is preferable to homogenize the suspension or the diluted suspension (hereunder, simply referred to as the "suspension") and then to dispense and add a part of it into the reaction solution. By performing such a homogenizing treatment in advance, it is possible to suppress the variance in the amount of nucleic acids added from the suspension to the reaction solution. Thus, more reproducible and reliable detection results can be obtained. The homogenizing treatment of the suspension is not particularly limited as long as the treatment is capable of sufficiently dispersing the solid components in the suspension, and can be exemplified by an agitation operation using a vortex mixer, a pipetting operation using a pipette, or the like.

The volume of the suspension to add to the reaction solution of the reverse transcription reaction or the nucleic acid amplification reaction can be appropriately set within a range such that the influence on the respective reaction by the carry-over of the inhibitory substances and the water-soluble organic solvent coming with nucleic acids from the suspension can be suppressed to a negligible level. For example, when an alcohol such as ethanol or a ketone such as acetone is used as the water-soluble organic solvent of the nucleic acid stabilizer solution, it is preferable to adjust the volume of the suspension to add to the reaction solution so that the alcohol concentration or the ketone concentration in the reaction solution becomes 1% or lower. When both an alcohol and a ketone are used, it is preferable to adjust the volume of the suspension to add to the reaction solution so that the total concentration of the alcohol and the ketone in the reaction solution becomes 1% or lower. Moreover, when an aldehyde such as formaldehyde is used as the water-soluble organic solvent, it is preferable to adjust the volume of the suspension to add to the reaction solution so that the aldehyde concentration in the reaction solution becomes 0.01% or lower.

Both DNA and RNA should be contained in the suspension. For example, the target nucleic acid can be detected by performing a nucleic acid amplification reaction such as PCR with use of DNA in the suspension as a template. Likewise, the target nucleic acid can be detected by performing a nucleic acid amplification reaction such as NASBA with use of RNA in the suspension as a template. In addition, the target nucleic acid can be detected by performing a reverse transcription reaction with use of RNA in the suspension as a template, and thereafter performing a nucleic acid amplification reaction with use of the obtained cDNA as a template. The cDNA obtained from the reverse transcription reaction may also be supplied to another known nucleic acid analysis method differing from the nucleic acid amplification reaction, so as to detect the target nucleic acid. The reverse transcription reaction and the nucleic acid amplification reaction can be performed by known methods in the art.

In cases where DNA in the suspension is the target of detection, for example, DNA mutation analyses or analyses of epigenetic changes can be performed by adopting nucleic acid amplification reactions. The mutation analyses can be exemplified by analyses of nucleotide insertion, deletion, substitution, duplication, inversion, or the like. Moreover, the analyses of epigenetic changes can be exemplified by analyses of methylation, demethylation, or the like.
Furthermore, it is also possible to check the presence or absence of the onset of cancer by detecting the presence or absence of genetic mutation(s) of a nucleotide sequence region including a microsatellite, or the like. On the other hand, in cases where RNA in the suspension is the target of detection, for example, it is possible to detect mutations, such as RNA nucleotide insertion, deletion, substitution, duplication, inversion, or splicing variants (isoforms). Alternatively, analyses of functional RNA (noncoding RNA), which can be exemplified by analyses of transfer RNA (tRNA), ribosomal RNA (rRNA), and microRNA (miRNA), can be carried out. Besides, it is also possible to detect and analyze the RNA expression level. It is particularly preferable to perform an mRNA expression analysis, a *K-ras* gene mutation analysis, a DNA methylation analysis, or the like. These analyses can be carried out by known methods in the art.

In the method for detecting a nucleic acid of the present invention, it is preferable to detect a nucleic acid derived from a mammalian cell, although it is also possible to detect a nucleic acid derived from any kind of biological species contained in the biological sample corresponding to the purpose. Moreover, when feces are used as a biological sample, it is preferable to detect a nucleic acid derived from a digestive tract cell or an exfoliated large intestine cell of a mammalian animal which excreted the feces.

In the detection of the nucleic acid in the step (C), it is particularly preferable to detect a marker for indicating neoplastic transformation or a marker for indicating an inflammatory digestive organ disease. Examples of such a marker for indicating neoplastic transformation can include known cancer markers such as the carcinoembryonic antigen (CEA) and the sialyl Tn (STN) antigen, and the presence or absence of mutation(s) in the *APC* gene, the *p53* gene, the *K-ras* gene, and the like. Moreover, the detection of methylation *of p16, hMLHI, MGMT, p14, APC, E-cadherin, ESR1, SFRP2,* or such genes is also useful as a diagnosis marker for colonic diseases (for example, refer to Lind et al., "A CpG island hypermethylation profile of primary colorectal carcinomas and colon cancer cell lines", Molecular Cancer, 2004, Vol. 3, Chapter 28). In addition, it is already reported that *Helicobacter pylori*-derived DNA in a fecal sample can be used as a marker for gastric cancer (for example, refer to Nilsson et al., Journal of Clinical Microbiology, 2004, Vol. 42, No. 8, pp. 3781-8). On the other hand, as a marker for an indicating inflammatory digestive organ disease, a nucleic acid derived from the *Cox-2* gene and the like can be exemplified. Note that, the nucleic acids derived from the *Cox-2* gene can also be used as a marker for indicating neoplastic transformation. For example, it is possible, by adopting the method for detecting a nucleic acid of the present invention, to employ feces as a biological sample so as to detect a nucleic acid derived from the *Cox-2* gene in the nucleic acid detection of the step (C). The thus obtained detection results can be applied to the detection of colon cancer.

### Examples

Next is a more detailed description of the present invention with reference to Examples. However, the present invention is not to be limited to the Examples below. Note that the symbol "%" refers to "volume %", unless otherwise specified. In addition, Caco-2 cells and HeLa cells serving as cultured cells were cultured by usual methods.

### Example 1

Using colon cancer patient artificial feces as a biological sample, nucleic acids derived from the *MDR1* (multidrug resistance 1) gene contained in the colon cancer patient artificial feces were detected by the method for detecting a nucleic acid of the present invention.
The colon cancer patient artificial feces were prepared by mixing 0.5 g of feces of a healthy subject with 5.0 × 10⁵ human colon carcinoma-derived cultured cells being Caco-2 cells, that were highly expressing the *MDR1* (multidrug resistance 1) gene.
Moreover, a 70% ethanol solution which had been adjusted to be acidic (pH 5.0) by a buffer solution of an acetic acid/sodium acetate buffer system was used as a nucleic acid stabilizer solution.

Specifically speaking, the colon cancer patient artificial feces were dispensed at 0.5 g each into two 15 mL polypropylene tubes, and 10 mL of the nucleic acid stabilizer solution was respectively mixed therein to prepare suspensions. The thus prepared suspensions were preserved in a thermostatic incubator at room temperature (25°C) for three days.
After the preservation, the suspension in one of these two polypropylene tubes was used for the preparation of a template solution of a reverse transcription reaction (RT template solution) by the preparation method 1 described below. The suspension in the other tube was used for the preparation of an RT template solution by the preparation method 2 described below. In other words, the RT template solution was prepared without extracting or purifying RNA from the fecal sample in either preparation method.

Preparation Method 1:
   The suspension was subjected to centrifugal separation at 15000 rpm for 10 minutes, and the majority of the supernatant was removed. Thereafter, the suspended matter containing the sedimentation was dispensed at 1 µL ten times, and each aliquot was used as an RT template solution.
Preparation Method 2:
   The suspension was not subjected to centrifugal separation but mixed by vortexing for 30 seconds. The thus homogenized suspension was dispensed at 1 µL ten times, and each aliquot was used as an RT template solution.

The total of twenty types of RT template solutions obtained from these two kinds of preparation methods were respectively used as a template to perform the RT reaction, and the thereby obtained cDNAs were used as a template to perform PCR. By so doing, an attempt was made to detect mRNA serving as a transcriptional product of the *MDR1* gene.
In the RT reaction, using the SuperScript (registered trademark) First-Strand Synthesis System for RT-PCR (a product of Invitrogen Corp.), 1 µL of the RT template solution was subjected to reverse transcription into cDNA. Specifically speaking, the RT reaction was performed by preparing an RT reaction solution having the composition shown in Table 2 and subjecting the solution to heat treatments at 25°C for 10 minutes, next at 42°C for 50 minutes, and thereafter at 70°C for 15 minutes.

**[Table 2]**

| | |
|---|---|
| RT template solution | 1 µL |
| Random hexamers | 1 µL |
| 10 mM DNTP mix | 1 µL |
| 10× RT buffer | 2 µL |
| 25 mM MgCl₂ | 4 µL |
| 0.1 M DTT | 2 µL |
| RNase OUT | 1 µL |
| SuperScript II | 1 µL |
| DEPEC-treated water | 7 µL |

After the RT reaction, 1 µL out of 20 µL of the solution was used as a template of the real-time PCR to detect the human *MDR1* gene. The MDR1 primer probe MIX of Applied Biosystems (catalog No: Hs00184500_ml) was used as the PCR primers. Specifically speaking, the obtained cDNA was dispensed at 1 µL in each well of a 0.2 mL 96-well PCR plate. Thereafter, in each well, 8 µL of extrapure water and 10 µL of a reagent for nucleic acid amplification (nucleotide chain elongation), "TaqMan GeneExpression Master Mix" (a product of Applied Biosystems), were added, and furthermore, 1 µL of the MDR1 primer probe MIX (a product of Applied Biosystems) was respectively added The mixture was respectively mixed. By so doing, the PCR reaction solution was prepared. The PCR plate was set in the ABI real-time PCR instrument, treated at 95°C for 10 minutes, and thereafter subjected to 40 thermal cycles, each cycle consisting of 95°C for 15 seconds and 60°C for 1 minute. The PCR was performed while measuring the fluorescence intensity per each cycle. The expression level of the *MDR1* gene in each fecal sample was assayed through analysis of the measurement results of the fluorescence intensity. An MDR1 plasmid was used for the calibration curve to determine the expression level.

**[Table 3]**

| | Preparation Method 1 | Preparation Method 2 |
|---|---|---|
| 1 | 28148 | 6775 |
| 2 | 9580 | 4564 |
| 3 | 840 | 5003 |
| 4 | 15721 | 4904 |
| 5 | 7034 | 4954 |
| 6 | 156 | 5384 |
| 7 | 4061 | 4430 |
| 8 | 23999 | 5057 |
| 9 | 13088 | 4874 |
| 10 | 102 | 4677 |
| Average | 10273 | 5062 |
| SD | 9943 | 659 |
| CV % | 97 | 13 |

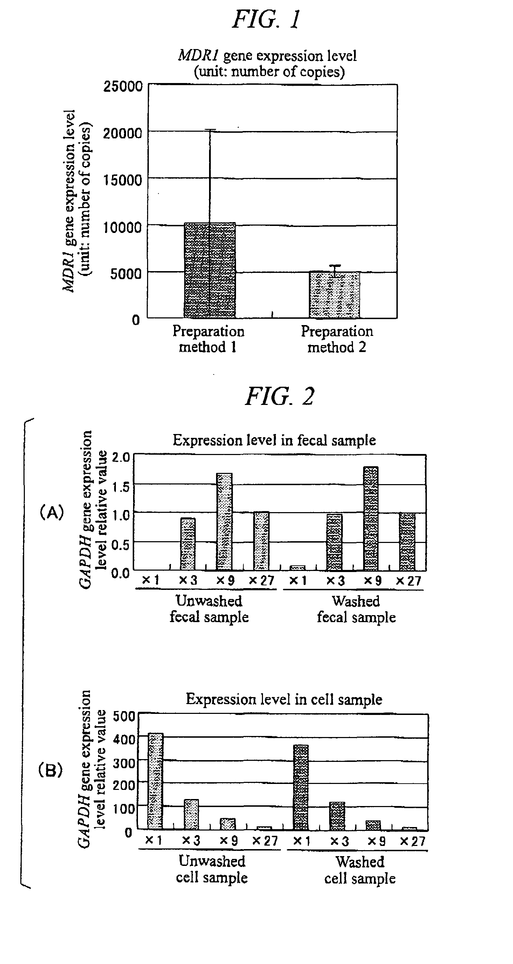

The assay results of the *MDR1* gene expression level are shown in Table 3 and FIG. 1. With the RT template solution prepared by the preparation method 1, the average values of the expression level were high but varied a lot, meaning that stable results were not obtained. In contrast, with the RT template solution prepared by the preparation method 2, the expression level varied a little, meaning that highly precise results were obtained.
The reason can be considered such that, because the centrifuged sedimentation was used as a template in the preparation method 1, the amount of the template was larger due to the thickening through centrifugation than that of the preparation method 2; whereas, however, the expression level was not stably obtainable because the sedimentation was non-homogeneous. In the preparation method 2, the reason why the uniform results were obtained was considered to be that the homogeneously dispersed suspension was used as a template.
The reproducibility is important not only in gene tests but also in clinical tests. In the preparation method 1, since results varied a lot, erroneous determination may be sometimes given to the test results. Therefore, highly reproducible preparation methods such as the preparation method 2, that is to say, such as the method employed in the method for detecting a nucleic acid of the present invention, are deemed to be useful.

### Example 2

Feces including lots of inhibitory substances and a culture product of cultured cells including almost no inhibitory substances were used as biological samples for the comparison to understand the influence of inhibitory substances on the nucleic acid detection.
A 70% ethanol solution (pH 5.5) was used as a nucleic acid stabilizer solution.
As a fecal sample, feces collected from a healthy subject were dispensed at 1 g each into two 15 mL polypropylene tubes, and 10 mL of the nucleic acid stabilizer solution was respectively mixed with each feces to prepare suspensions.
On the other hand, as a cell sample, 1 × 10⁶ HeLa cells cultured by a usual method were respectively dispensed into two 15 mL polypropylene tubes, and 10 mL of the nucleic acid stabilizer solution was respectively mixed with each cell solution to prepare suspensions.
The respective samples were preserved at 25°C for one day. Then, one of each sample from both the fecal and cell samples was subjected to centrifugal separation at 2000 rpm for 10 minutes, and the supernatant was removed. Thereafter, the obtained sedimentation was respectively mixed with 10 mL of 0.1M citric acid/NaOH buffer (pH 5.0). By so doing, washed suspensions were prepared.
The thus prepared four types of suspensions (unwashed fecal sample, washed fecal sample, unwashed cell sample, and washed cell sample) were used as the assay samples. Each suspension was diluted in four three-fold steps so that suspensions having dilution factors from × 1 to x27 were prepared. These were used as RT template solutions.
The RT reaction was performed using 1 µL of each RT template solution in the same manner as that of Example 1. After the RT reaction, 1 µL out of 20 µL of the solution was used as a template of the real-time PCR. The real-time RT-PCR was performed by using 1 µL of the GAPDH primer probe MIX (a product of Applied Biosystems, catalog No: Hs02786624_gl) in the same manner as that of Example I to detect the human *GAPDH* gene.

The measurement results of the fluorescence intensity were analyzed and the relative values of the *GAPDH* gene expression level were calculated by assuming that the expression level of the 27-fold dilution solution of the washed fecal sample (in FIG. 2(A), referred to as the "washed fecal sample x27") was 1 as the standard of the relative value of the expression level. The calculation results are shown in FIG. 2. FIG. 2 (A) shows the results of cases where the diluted suspensions of the unwashed fecal sample and the washed fecal sample were used as the RT template solution. FIG. 2 (B) shows the results of cases where the diluted suspensions of the unwashed cell sample and the washed cell sample were used as the RT template solution.
As shown in FIG. 2 (A), in both cases of the unwashed fecal sample and the washed fecal sample, the *GAPDH* gene expression level increased as the dilution factor went higher until the dilution solution was up to 9-fold (x9). This can be considered such that, as the dilution factor went higher, the amount of the carry-out of inhibitory substances from the fecal sample to the RT reaction solution and to the following PCR reaction solution decreased, and therefore an increase in the expression level was observed although the amount of nucleic acids added to the reaction solution was reduced. When using the RT template solution of 1-fold dilution (×1), the amount of inhibitory substances in the RT reaction solution was one twentieth of that of the RT template solution, since the volume of the RT template solution was 1 µL and the total volume of the RT reaction solution was 20 µL; however, nonetheless, inhibition of the RT reaction was found. On the other hand, with the 27-fold dilution solution (x27), the amount of the carry-over of inhibitory substances was so sufficiently reduced that their inhibitory actions were alleviated, and therefore the expression level decreased by reflecting the reduction in the amount of the template due to dilution.
On the other hand, when it comes to the cell sample, as shown in FIG. 2 (B), the *GAPDH* gene expression level decreased as the dilution factor went higher, in both cases of the unwashed cell sample and the washed cell sample. This suggests that, unlike the fecal sample, the reaction occurred in the cell sample without receiving any inhibition, and therefore the expression level was dependent on the amount of nucleic acids serving as the template added to the reaction system.
From these results, it is apparently possible to detect a nucleic acid in a biological sample not by extracting or purifying nucleic acids, but by appropriately diluting a prepared suspension, even if a biological sample such as feces which contains lots of inhibitory substances is used.

### Reference Example 1

Purified nucleic acids were used as a template to investigate the influence of water-soluble organic solvent (ethanol, acetone, and formaldehyde) mixed in the reaction solution, on the RT reaction and PCR.
Using the RNeasy mini kit (a product of QIAGEN), RNA was extracted from HeLa cells that had been cultured by a usual method. This sample was used as template RNA.
The RT reaction was performed by using the high capacity RNA-to-cDNA kit (a product of Applied Biosystems). The RT reaction solutions were prepared so that the final concentration of the organic solvent became 1, 5, 10, or 30% ethanol, 1, 5, 10, or 30% acetone, or 0.01, 0.1, 1, or 10% formaldehyde.
As a control, an RT reaction solution not including any water-soluble organic solvent was also prepared.
Specifically speaking, 1 µL of template RNA, 10 µL of 2×RT buffer, 1 µL of 20x Enzyme MIX, and 8 µL of purified water containing each water-soluble organic solvent were mixed in a 0.2 mL PCR tube, which was then subjected to heat treatments at 37°C for 60 minutes and next at 95°C for 5 minutes. By so doing, cDNA was synthesized. Then, after the RT reaction, I µL out of 20 µL of the solution was used as a template of the real-time PCR. The real-time RT-PCR was performed by using 1 µL of the GAPDH primer probe MIX (a product of Applied Biosystems, catalog No: Hs02786624_gl) in the same manner as that of Example 1 to detect the human *GAPDH* gene.

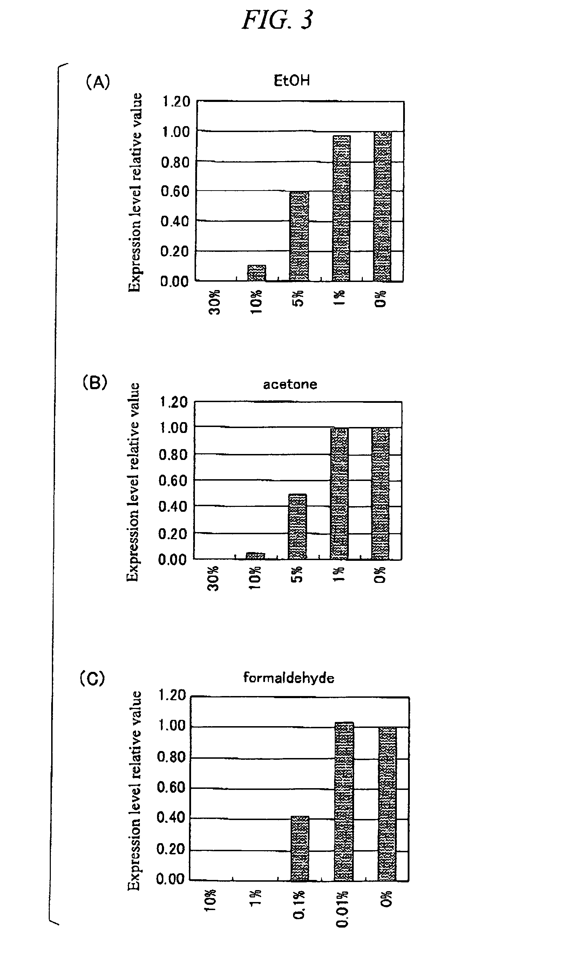

The measurement results of the fluorescence intensity were analyzed and the relative values of the *GAPDH* gene expression level were calculated by assuming that the result of the RT reaction solution not including any water-soluble organic solvent was 1 as the standard of the relative value of the expression level. The calculation results are shown in FIG. 3. FIG. 3 (A) shows the results of cases where ethanol was mixed. FIG. 3 (B) shows the results of cases where acetone was mixed. FIG. 3 (C) shows the results of cases where formaldehyde was mixed. In these graphs, the symbol "X%" refers to the proportion of each water-soluble organic solvent mixed therein.
As shown in FIGs. 3 (A) and (B), in cases where ethanol or acetone was mixed in the RT reaction solution, the expression level was not obtained at all when the final concentration was 30%. This suggests that the reaction was inhibited. In addition, the expression level increased as the concentration decreased from 10%. When the concentration was 1%, the expression level became equivalent to that of 0% concentration (including no water-soluble organic solvent), and any influence (inhibitory action) of the mixed water-soluble organic solvent was not seen at all.
On the other hand, as shown in FIG. 3 (C), when it comes to formaldehyde, the expression level was obtained when the proportion of formaldehyde mixed in the RT reaction solution was 0.1 % or lower. When the proportion was 0.01%, any influence (inhibitory action) of the mixed water-soluble organic solvent was not seen at all.

In other words, it can be considered to be preferable that the volume of the suspension to add is determined by considering the final concentration of the water-soluble organic solvent in the RT reaction solution if a suspension of a biological sample and a nucleic acid stabilizer solution is used in the RT reaction, and particularly preferable that the volume of the suspension to add is determined so that the final concentration of ethanol or acetone in the RT reaction solution becomes 10% or lower, preferably 5% or lower, and more preferably 1% or lower, or the final concentration of formaldehyde becomes 0.1% or lower, and preferably 0.01% or lower.

### Industrial Applicability

With the method for detecting a nucleic acid of the present invention, a nucleic acid from a biological sample such as feces can be detected with sufficient precision without a need for complicated manipulations. Therefore, the method is particularly applicable to the fields of clinical tests for routine checkups or the like where biological samples are used.

## Claims

1. A method for detecting a nucleic acid in a biological sample, comprising:
(A) mixing a biological sample with a nucleic acid stabilizer solution comprising a water-soluble organic solvent as an active ingredient, and then preserving the thus yielded suspension for a predetermined period of time; and
(B) performing a reverse transcription reaction and/or a nucleic acid amplification reaction with use of a part of the suspension yielded in (A) as a template solution, thereby detecting a nucleic acid in the suspension.

2. The method for detecting a nucleic acid in a biological sample according to Claim 1, wherein the suspension yielded in (A) is diluted and then a part of the diluted suspension is used as a template solution in (B).

3. The method for detecting a nucleic acid in a biological sample according to Claim 1 or 2, wherein said nucleic acid stabilizer solution includes one or more types selected from the group consisting of a water-soluble alcohol, a ketone, and an aldehyde, as said water-soluble organic solvent.

4. The method for detecting a nucleic acid in a biological sample according to Claim 3, wherein said water-soluble organic solvent includes one or more types selected from the group consisting of ethanol, propanol, and methanol, as said water-soluble alcohol.

5. The method for detecting a nucleic acid in a biological sample according to Claim 3, wherein said water-soluble organic solvent includes acetone and/or methyl ethyl ketone, as said ketone.

6. The method for detecting a nucleic acid in a biological sample according to any one of Claims 1 to 5, wherein the pH of said nucleic acid stabilizer solution is from 2 to 6.5.

7. The method for detecting a nucleic acid in a biological sample according to any one of Claims 1 to 6, wherein the period of time for preserving said suspension in (A) is one hour or longer.

8. The method for detecting a nucleic acid in a biological sample according to any one of Claims 2 to 7, wherein said suspension is diluted in (B) by adding any one or more types selected from the group consisting of an acidic buffer solution whose pH is 2 or higher, said nucleic acid stabilizer solution, and purified water.

9. The method for detecting a nucleic acid in a biological sample according to Claim 8, wherein said acidic buffer solution is a buffer solution selected from the group consisting of an acetic acid/sodium acetate buffer system, a citric acid/sodium hydroxide buffer system, and a lactic acid/sodium lactate buffer system.

10. The method for detecting a nucleic acid in a biological sample according to any one of Claims 1 to 9, wherein said nucleic acid stabilizer solution further includes one or more types selected from the group consisting of a protease inhibitor and a polycation.

11. The method for detecting a nucleic acid in a biological sample according to any one of Claims 1 to 10, wherein said biological sample is feces, blood, or urine.

12. The method for detecting a nucleic acid in a biological sample according to any one of Claims 1 to 11, wherein the method is to detect a nucleic acid derived from a mammalian cell contained in said biological sample.

13. The method for detecting a nucleic acid in a biological sample according to Claim 12, wherein said mammalian cell is a gastrointestinal tract cell or an exfoliated large intestine cell.

14. The method for detecting a nucleic acid in a biological sample according to Claim 12 or 13, wherein said nucleic acid derived from a mammalian cell is a marker for neoplastic transformation or a marker for an inflammatory gastrointestinal tract disease.

15. The method for detecting a nucleic acid in a biological sample according to Claim 12 or 13, wherein said nucleic acid derived from a mammalian cell is a nucleic acid derived from a *Cox-2* gene.
